(19) Europäisches Patentamt
European Patent Office
Office européen des brevets

(11) **EP 1 371 977 B1**

(12) **FASCICULE DE BREVET EUROPEEN**

(45) Date de publication et mention de la délivrance du brevet:
**26.10.2011 Bulletin 2011/43**

(51) Int Cl.:
*G01N 33/15* (2006.01)  *G01N 21/55* (2006.01)
*G01N 21/47* (2006.01)  *G01N 21/17* (2006.01)
*A61B 5/103* (2006.01)

(21) Numéro de dépôt: **03291364.2**

(22) Date de dépôt: **06.06.2003**

(54) **Procédé pour déterminer l'aptitude à diffuser et/ou à absorber la lumière d'un produit cosmétique ou dermatologique**

Verfahren zur Ermittlung der Lichtstreuungs- und/oder Lichtabsorptionsfähigkeit eines kosmetischen oder dermatologischen Produkts

Method for determining the aptitude of a cosmetic or dermatological product at diffusing and/or absorbing light

(84) Etats contractants désignés:
**DE ES FR GB IT**

(30) Priorité: **10.06.2002 FR 0207108**

(43) Date de publication de la demande:
**17.12.2003 Bulletin 2003/51**

(73) Titulaire: **L'Oréal**
**75008 Paris (FR)**

(72) Inventeurs:
• **Querleux, Bernard**
**94170 Le Perreux (FR)**
• **Saint-Jalmes, Hervé**
**69004 Lyon (FR)**

(74) Mandataire: **Tanty, François et al**
**Nony & Associés**
**3, rue de Penthièvre**
**75008 Paris (FR)**

(56) Documents cités:
**EP-A- 0 993 601     EP-A- 1 156 074**
**WO-A-95/05892     WO-A-02/057726**
**DE-U- 29 719 497     US-A- 5 781 289**
**US-A- 5 923 039**

• **PATENT ABSTRACTS OF JAPAN vol. 1995, no. 10, 30 novembre 1995 (1995-11-30) & JP 07 167781 A (SHISEIDO CO LTD), 4 juillet 1995 (1995-07-04)**

## Description

La présente invention concerne un procédé pour la détermination de grandeurs caractéristiques du comportement optique d'une surface, notamment de peau et/ou de fibres kératiniques sur laquelle ou lesquelles a été appliqué un produit cosmétique, et un dispositif pour sa mise en oeuvre.

On entend par produit cosmétique tout produit tel que défini dans la Directive 76/768/CEE modifiée par la Directive 93/35/CEE du 14 juin 1993.

L'article « Integrating the digitized backscattered image to measure absorption and reduced-scattering coefficients *in vivo"*, Gobin *et al.,* publié dans la revue APPLIED OPTICS du 1er juillet 1999, fait état de mesures de coefficients de diffusion et d'absorption effectuées en laboratoire sur la peau nue.

La demande de brevet EP 1 156 074 A décrit un procédé pour évaluer un produit de protection solaire, dans lequel une peau artificielle imprégnée de ce produit est éclairée par un rayonnement UV et dans lequel le rayonnement UV transmis à travers la peau artificielle est mesurée par un spectrophotomètre.

Le brevet US 5 923 039 décrit un procédé pour analyser la lumière ultraviolette transmise à travers un échantillon illuminé par un rayonnement ultraviolet (UV), au moyen d'un ensemble de photodiodes, chaque photodiode mesurant une brande étroite du spectre UV, de façon à déterminer la dispersion de la lumière ultraviolette transmise par l'échantillon.

L'invention a pour objet, selon l'un de ses aspects, un procédé pour déterminer l'aptitude à diffuser et/ou à absorber la lumière d'un produit cosmétique ou dermatologique éventuellement appliqué sur un support, ce procédé étant caractérisé par le fait qu'il comporte les étapes suivantes :

 a) éclairer le produit et/ou une zone du support sur laquelle a été appliqué le produit avec un faisceau lumineux incident de manière à former une tache de rétrodiffusion,
 b) acquérir une image de la tache, et
 c) analyser l'image pour déterminer, en fonction de la variation de la luminosité en différents points de la tache, au moins une information représentative de l'aptitude à diffuser la lumière et/ou à l'absorber du produit et/ou du support en présence du produit.

Le procédé peut exclure tout traitement thérapeutique du corps humain ou animal.

Le produit illuminé par le faisceau lumineux incident peut former par exemple une masse relativement épaisse, étant par exemple contenu dans un récipient. Il peut également former une couche à la surface d'un support, une telle couche pouvant être relativement peu épaisse. Le produit peut encore avoir été absorbé par un support.

Dans un exemple de mise en oeuvre de l'invention, on peut ainsi déterminer, en fonction de la variation de la luminosité en différents points de la tache, notamment en des points situés à des distances différentes du centre de la tache, un coefficient de diffusion réduit $\mu'_s$. On peut également déterminer, en variante ou additionnellement, un coefficient d'absorption $\mu_a$.

Le support peut comporter des fibres et/ou des cellules kératiniques. Ce support peut être inerte ou vivant.

La surface éclairée par le faisceau lumineux incident peut être sensiblement plane ou présenter des reliefs.

Le produit peut avoir été appliqué sur le support sur une superficie occupant, par exemple, entre 0,5 cm$^2$ et 5 cm$^2$, notamment entre 1 cm$^2$ et 2 cm$^2$.

Le faisceau lumineux incident peut être un faisceau de lumière blanche ou, en variante, un faisceau de lumière monochromatique, notamment visible, par exemple de couleur rouge ou bleue. La longueur d'onde peut être choisie en fonction, par exemple, de la nature du support et/ou des propriétés optiques que l'on cherche à déterminer. Le faisceau lumineux peut être un faisceau de lumière cohérente.

La section du faisceau lumineux peut être inférieure ou égale à 4 $\mu$m, de préférence inférieure à 2 $\mu$m.

L'intensité du faisceau lumineux incident est de préférence sensiblement constante sur toute sa section. Le faisceau lumineux incident étant produit par une source lumineuse, un filtre spatial peut être disposé entre la source et le produit.

L'angle d'incidence du faisceau lumineux incident avec la normale à la surface du produit peut être compris entre 5˚ et 25˚ par exemple, notamment entre 10˚ et 20˚.

Un miroir peut, le cas échéant, être disposé sur le trajet du faisceau lumineux incident.

Avantageusement, un dispositif sans miroir peut être utilisé pour les mesures *in vivo*.

On peut acquérir l'image de diverses façons, par exemple au moyen d'une caméra, notamment une caméra CCD monochrome. Le terme « caméra » englobe non seulement les caméras destinées à l'acquisition d'une séquence d'images, mais également les appareils photographiques numériques.

Le traitement des données peut s'effectuer localement, mais en variante on peut transmettre à distance, notamment par le réseau Internet, l'image acquise à un centre de traitement, afin de l'analyser.

L'acquisition de l'image peut s'effectuer au travers d'un système optique ayant un grossissement variable. Cela permet d'agrandir l'image pour qu'elle occupe une majorité du champ de prise de vue, de manière à bénéficier de la meilleure résolution.

Un polariseur peut être disposé sur le trajet du faisceau lumineux incident, entre la source et le produit, et l'image peut être acquise au travers d'un analyseur, disposé entre le produit et la caméra.

La tache peut être observée en l'absence de toute lumière parasite, mais le faisceau lumineux incident peut être modulé et l'acquisition de l'image être synchrone, ce qui peut permettre d'effectuer des mesures en présence de lumière parasite.

Le produit peut être appliqué sur le support avec une épaisseur, notamment sensiblement constante, comprise entre 7 µm et 20 µm, par exemple.

Le produit peut présenter une composition homogène.

Le produit peut être choisi par exemple parmi les produits suivants : fond de teint, filtre solaire, produit dépigmentant, produit anti-rides, produit hydratant, cette liste n'étant pas limitative.

Le produit peut être apte à modifier au moins une propriété physico-chimique du support sur lequel il est appliqué, ce qui est le cas par exemple d'un produit dépigmentant.

L'invention a encore pour objet, selon un autre de ses aspects, un procédé pour déterminer une caractéristique d'un produit cosmétique, dans lequel :

- on détermine l'aptitude à diffuser et/ou à absorber la lumière de deux surfaces composées respectivement,

    a) du produit appliqué sur la peau et/ou les fibres kératiniques, et
    b) de la peau et/ou des fibres kératiniques en l'absence de produit,

en réalisant au moins pour chacune les étapes suivantes :

    i) éclairer une zone de la surface considérée avec un faisceau lumineux incident de manière à former une tache de rétrodiffusion,
    ii) acquérir une image de la tache,
    iii) analyser l'image pour déterminer, en fonction de la variation de la luminosité en différents points de la tache, au moins une information représentative de l'aptitude du produit, de la peau et/ou des fibres kératiniques à diffuser la lumière et/ou à l'absorber, et dans lequel

- on détermine en fonction des informations obtenues pour la peau et/ou les fibres kératiniques avec et sans produit au moins une caractéristique du produit.

Le procédé peut exclure tout traitement thérapeutique du corps humain ou animal.

La caractéristique du produit qui est ainsi déterminée peut être sa couvrance ou sa tenue.

Lorsque le produit est un filtre solaire, la caractéristique qui est déterminée peut être l'efficacité de la protection conférée par le produit.

Dans le cas d'un produit dépigmentant, la caractéristique qui est déterminée peut être l'efficacité du produit.

L'invention a encore pour objet, selon un autre de ses aspects, un procédé pour déterminer l'évolution dans le temps d'une caractéristique d'un produit cosmétique, caractérisé par le fait qu'il comporte les étapes suivantes :

    a) appliquer le produit sur un support,
    b) éclairer une zone du support sur laquelle le produit a été appliqué avec un faisceau lumineux incident de manière à former une tache de rétrodiffusion,
    c) acquérir une première image de la tache,
    d) analyser la première image pour déterminer, en fonction de la variation de la luminosité en différents points de la tache, au moins une information représentative de l'aptitude du produit à diffuser la lumière et/ou à l'absorber, à un premier instant,
    e) acquérir une seconde image de la tache, à un second instant,
    f) analyser la seconde image pour déterminer, en fonction de la variation de la luminosité en différents points de la tache, au moins une information représentative de l'aptitude du produit à diffuser la lumière et/ou à l'absorber, à ce second instant,
    g) déterminer en fonction des informations obtenues lors des étapes d) et f) l'évolution dans le temps d'une caractéristique du produit, notamment l'une de celles précitées.

Le procédé peut exclure tout traitement thérapeutique du corps humain ou animal.

Dans un exemple de mise en oeuvre de l'invention, le procédé peut comporter l'étape suivante, entre les étapes d) et e) :

- exercer une action sur le produit et/ou le support.

Cette action peut être par exemple une action de lavage du support. Cela peut permettre par exemple de déterminer la résistance à l'eau d'un produit solaire ou de maquillage. L'action exercée peut encore être autre, par exemple une exposition au vent ou au rayonnement ultraviolet ou un contact avec un élément sur lequel le produit est susceptible de se transférer.

L'invention a encore pour objet, selon un autre de ses aspects, un procédé de prescription ou de fabrication d'un produit cosmétique, caractérisé par le fait qu'il comporte les étapes suivantes :

    a) éclairer une zone de peau avec un faisceau lumineux incident de manière à former une tache de rétrodiffusion,
    b) acquérir une image de la tache,
    c) analyser l'image pour déterminer, en fonction de la variation de la luminosité en différents points de la tache, au moins une information représentative de l'aptitude de la peau à diffuser la lumière et/ou à l'absorber,

d) prescrire ou fabriquer, au moins en fonction de cette information, un produit cosmétique ayant une aptitude à diffuser la lumière et/ou à l'absorber proche de celle précédemment déterminée pour la peau.

Le produit cosmétique ainsi fabriqué ou prescrit peut être destiné par exemple à masquer des imperfections de la peau et à réaliser un maquillage naturel.

L'invention a encore pour objet un dispositif de détermination de l'aptitude d'un produit cosmétique à diffuser et/ou à absorber la lumière, pouvant se caractériser par le fait qu'il comporte

- une source apte à délivrer un faisceau lumineux capable de former une tache lumineuse sur le produit ou sur un support sur lequel a été appliqué un produit,
- une caméra, notamment une caméra CCD monochrome, permettant d'acquérir au moins une image de la tache,
- un dispositif d'analyse pour analyser cette image et déterminer, en fonction de la variation de la luminosité en différents points de la tache, notamment en différents points situés à des distances différentes du centre de la tache, au moins une information représentative de l'aptitude du produit à diffuser la lumière et/ou à l'absorber.

La source lumineuse peut comporter un laser.
Le dispositif peut comporter un filtre spatial traversé par le faisceau lumineux et, le cas échéant, un miroir placé sur le trajet du faisceau lumineux.
La source lumineuse peut être apte à émettre avec au moins deux longueurs d'onde différentes, simultanément ou non.
Le dispositif d'analyse peut être configuré pour recevoir l'image acquise par la caméra par un réseau, notamment le réseau Internet.
L'invention pourra être mieux comprise à la lecture de la description détaillée qui va suivre, d'exemples de mise en oeuvre non limitatifs, et à l'examen du dessin annexé, sur lequel :

- la figure 1 est une vue schématique et simplifiée d'un dispositif permettant de mettre en oeuvre un exemple de procédé selon l'invention,
- la figure 2 est un exemple d'image de tache de rétrodiffusion sur la peau après application d'un produit cosmétique,
- la figure 3 est un exemple d'image de tache de rétrodiffusion sur la peau avant application d'un produit cosmétique,
- la figure 4 est un graphique permettant de calculer les coefficients de diffusion réduit et d'absorption,
- la figure 5 est un exemple d'image résultant de la différence des images obtenues en lumière incidente polarisée horizontalement et lumière rétrodiffusée polarisée horizontalement ou verticalement, et
- la figure 6 est une vue schématique et simplifiée d'un autre dispositif permettant de mettre en oeuvre un exemple de procédé selon l'invention.

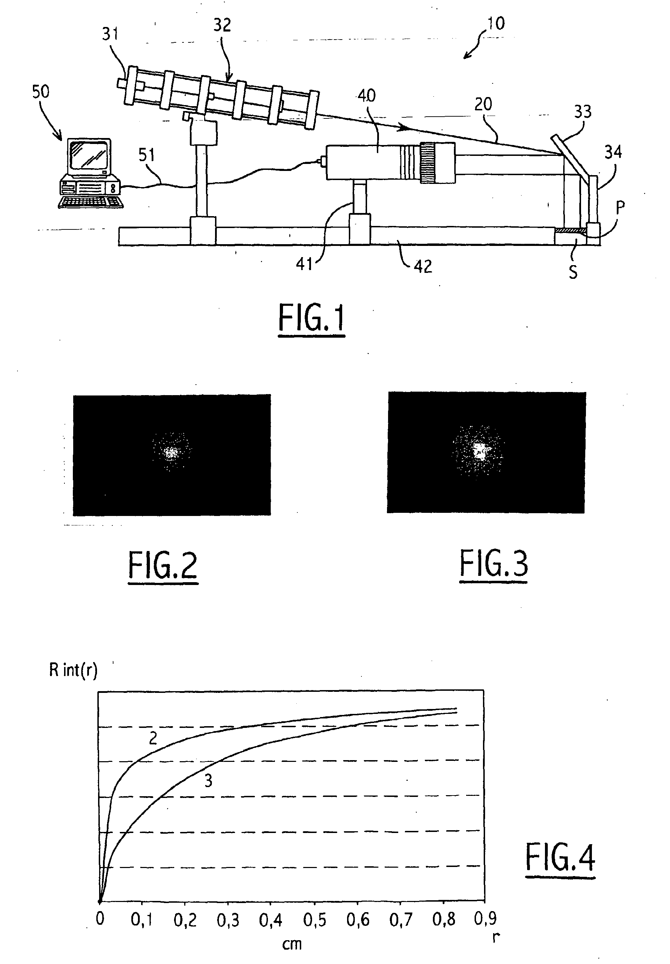

L'invention, selon l'un de ses aspects, permet la détermination d'au moins un coefficient de diffusion réduit $\mu'_s$ et/ou d'absorption $\mu_a$ de la lumière, par observation d'une surface sur laquelle a été appliqué un produit cosmétique.
Le coefficient de diffusion réduit $\mu'_s$ traduit le changement de la répartition spatiale d'un faisceau dévié dans de multiples directions par une surface ou par un milieu, sans changement de fréquences des rayonnements monochromatiques qui le composent.
Le coefficient d'absorption $\mu_a$ traduit la diminution de l'intensité du faisceau à la traversée d'une matière, l'énergie rayonnante étant transformée en une autre forme d'énergie.
On a représenté à la figure 1 un dispositif 10 permettant de déterminer une valeur représentative de l'aptitude d'un produit cosmétique P à diffuser la lumière et/ou à l'absorber. Le produit P est appliqué sur un support S, qui peut être, par exemple, un support inerte, de la peau humaine ou des fibres kératiniques, cette liste n'étant pas limitative.
Le produit P est appliqué, dans l'exemple illustré, sur le support S avec une épaisseur comprise entre 10 et 15 $\mu$m.
On éclaire une zone du support S revêtue du produit P avec un faisceau lumineux incident 20, pour former une tache de rétrodiffusion.
Le faisceau lumineux 20 est monochromatique, dans l'exemple considéré, étant délivré par un laser 31.
Dans l'exemple décrit, la longueur d'onde produite par le laser est de 635 nm environ, mais on ne sort pas du cadre de la présente invention lorsque le faisceau lumineux incident est produit par un autre type de source et présente une longueur d'onde différente, par exemple dans le bleu ou l'UV. On peut ainsi encore utiliser, par exemple, comme source de lumière un laser HeNe, une ou plusieurs diodes électroluminescentes ou une source de lumière blanche.
Le faisceau lumineux 20 traverse un filtre spatial 32 permettant d'obtenir une répartition homogène de l'intensité lumineuse du faisceau.
Dans l'exemple décrit, le support S sur lequel le produit P a été appliqué est placé horizontalement et le laser 31 est faiblement incliné par rapport à l'horizontale. Pour éclairer le support S avec un faisceau lumineux faiblement incliné par rapport à la normale, un miroir 33 est placé sur le trajet du faisceau incident 20, ce miroir étant incliné à 45˚ environ par rapport à la verticale. On peut modifier éventuellement l'orientation du faisceau lumineux incident en faisant varier l'inclinaison du miroir 33 par rapport au bâti 34.
On acquiert, au moyen d'une caméra 40, une image digitalisée de la tache formée par le faisceau lumineux, après réflexion dans le miroir 33, un exemple d'une telle image étant donné à la figure 2. Dans cet exemple, le produit est un fond de teint.

A la figure 3, on a donné un exemple d'une image digitalisée de la tache formée sur la peau avant application du produit.

Dans l'exemple décrit, la caméra est une caméra CCD monochrome ayant une résolution de 490 par 660 pixels, mais on ne sort pas du cadre de la présente invention en utilisant une caméra ayant une résolution différente, éventuellement couleur.

La caméra est fixée sur un pied 41 mobile sur un guide 42 parallèlement au support S, ce qui permet de régler la distance entre l'objectif et le support S.

Lorsqu'on éclaire le produit P appliqué sur le support S avec un faisceau lumineux de faible longueur d'onde, la tache de rétrodiffusion est relativement petite car il y a plus d'absorption.

On peut grossir dans ce cas l'image en diminuant la distance entre l'objectif et l'échantillon.

En variante, on peut munir la caméra d'un objectif à grossissement variable, permettant d'adapter la taille du champ de prise de vue à la taille de la tache de rétrodiffusion.

L'information recueillie par la caméra est transmise à un ordinateur 50 par un câble 51.

L'ordinateur 50 est configuré pour, en fonction de la luminosité de chaque pixel de l'image digitalisée, calculer des valeurs représentatives de l'aptitude du produit à diffuser la lumière et/ou à l'absorber. L'ordinateur 50 peut ainsi être programmé pour calculer des coefficients d'absorption et de diffusion.

La réflectance R(r, φ), qui est le rapport de l'intensité de la lumière réfléchie à l'intensité de la lumière incidente, peut être calculée en fonction des coordonnées cylindriques, à partir du centre de la tache.

En intégrant R (r, φ) en fonction de φ, on obtient la fonction R (r) :

$$\overline{R}(r) = \oint R(r,\varphi)\frac{d\varphi}{2\pi}$$

On a représenté à la figure 4 la valeur de la réflectance R de la tache intégrée en fonction du rayon r :

$$Rint(r) = \int_0^r R(\rho)\, 2\,\pi\,\rho\, d\,\rho,$$

On obtient une fonction du rayon dont les constantes permettent d'accéder aux coefficients de diffusion et d'absorption :

$$Rint(r) = a\left[1 - \exp\left(-\frac{r}{b}\right)\right]$$

et où $a_1$, $a_2$, $b_1$ et $b_2$ ne dépendent que de l'indice de réfraction n.

avec:

$$a = a_1 \exp\left[-a_2\left(1 + \frac{\mu'_s}{\mu_a}\right)^{-0,5}\right]$$

On obtient pour la courbe 2 correspondant à la tache de la figure 2 obtenue

$$b = \left[b_1 + b_2 \ln\left(\frac{\mu_a}{\mu'_s}\right)\right]\mu'_s{}^{-1}.$$

après application d'un fond de teint les valeurs $\mu_a$ = 2,82 $cm^{-1}$ et $\mu'_s$ = 94,72 $cm^{-1}$, et pour la courbe 3 correspondant à la tache de la figure 3 obtenue avant application du produit les valeurs : $\mu_a$ = 1,11 $cm^{-1}$ et $\mu'_s$ = 28,77 $cm^{-1}$.

L'image de la tache peut être soustraite à une image noire, afin d'annuler statistiquement une grande partie du bruit thermique.

On ne sort pas du cadre de la présente invention lorsqu'on place au moins un polariseur sur le trajet du faisceau lumineux incident, entre la source et le produit, et/ou de la lumière rétrodiffusée, entre le produit et la caméra.

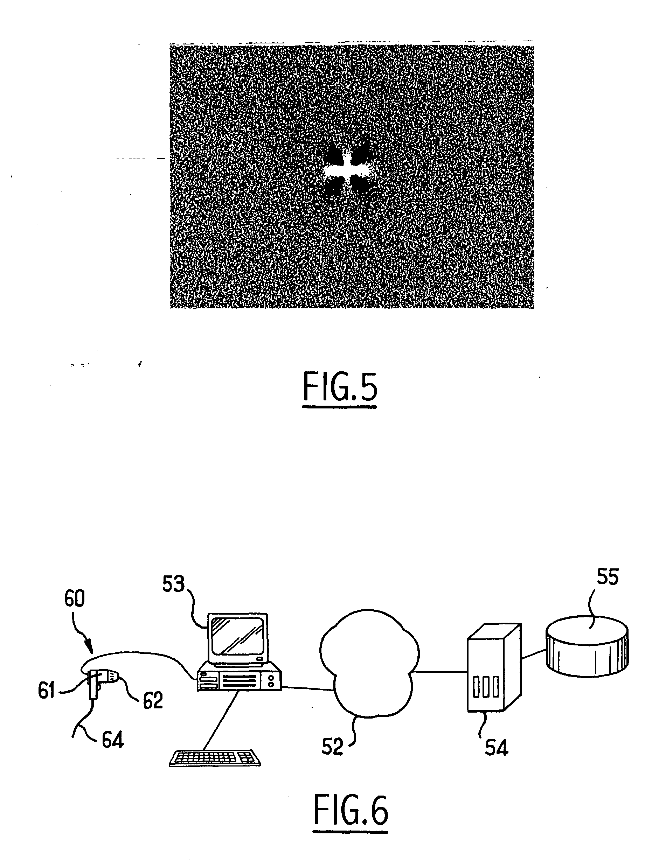

A titre d'exemple, on a représenté à la figure 5 une image normalisée résultant de la différence des images obtenues en polarisant horizontalement le faisceau lumineux incident et en polarisant horizontalement d'une part et/ou verticalement d'autre part la lumière rétrodiffusée.

Cette opération permet de calculer deux paramètres optiques supplémentaires, le coefficient d'anisotropie g et le coefficient de diffusion pure $\mu_s$.

Pour cela, on acquiert trois images : la première sans polarisation, le seconde en polarisant horizontalement les lumières incidente et rétrodiffusée, et la troisième en polarisant horizontalement la lumière incidente et verticalement la lumière rétrodiffusée.

La première image permet de calculer les coefficients d'absorption $\mu_a$ et de diffusion réduit $\mu'_s$, comme expliqué plus haut, et les deux autres de calculer, par le calcul de $\mu'_s$, le coefficient d'anisotropie g et $\mu_s$.

On ne sort pas du cadre de la présente invention lorsqu'on utilise un dispositif portatif pour acquérir l'image de la tache de rétrodiffusion.

A titre d'exemple, on a représenté sur la figure 6 un dispositif portatif 60 comportant une source de lumière et une caméra, par exemple du type webcam, placées dans un boîtier 61. Ce boîtier 61 comporte une ouverture 62 permettant la sortie du faisceau lumineux 20 provenant de la source de lumière et l'acquisition de l'image avec la caméra.

Les données saisies par la caméra peuvent être transmises au moyen d'un ordinateur 53 via un réseau 52, notamment le réseau Internet, à un centre de traitement 54 relié à une base de données 55 et configuré pour traiter les informations transmises et calculer les coefficients de diffusion réduit et d'absorption.

Le centre de traitement 54 peut en outre être programmé pour effectuer, au vu des données transmises et des calculs effectués, un diagnostic et préconiser un produit adapté, un soin ou un maquillage et des conseils.

Ce centre de traitement 54 peut également être agencé pour permettre de collecter différentes données afin de constituer, par exemple, une banque de données contenant des caractéristiques représentatives de l'aptitude de différents produits cosmétiques à diffuser et/ou à absorber la lumière.

Le dispositif portatif 60 peut être présent dans un institut ou sur un point de vente par exemple, et peut être utilisé en vue de délivrer un produit de maquillage et/ou de soins personnalisé, en particulier qui soit fonction du type de peau et/ou de fibres kératiniques du client.

Le produit dont on détermine des caractéristiques optiques peut être par exemple un fond de teint.

Dans ce cas, on peut calculer d'abord les coefficients de diffusion réduit et d'absorption de la peau nue. On applique ensuite le fond de teint sur la peau avant de mesurer les coefficients de diffusion réduit et d'absorption de la peau sur laquelle a été appliquée la couche de fond de teint. En comparant les valeurs obtenues avant et après l'application du fond de teint sur la peau, on peut obtenir une évaluation de la couvrance de ce fond de teint.

On peut également caractériser l'adéquation entre un fond de teint et la peau en procédant de manière similaire. On calcule les coefficients de diffusion réduit et d'absorption de la peau avant et après l'application du fond de teint, et on déduit de la comparaison de ces valeurs une information sur l'aptitude du fond de teint à conférer un maquillage naturel.

On peut encore, grâce à l'invention, préconiser un produit adapté à un type de peau ou de fibres kératiniques.

Pour cela, on peut enregistrer dans la banque de données 55 des valeurs caractéristiques de la diffusion et/ou de l'absorption de la lumière d'un ensemble de produits. On peut ensuite déterminer pour un individu, par exemple avec le dispositif 60, des valeurs caractéristiques de l'absorption et/ou de la diffusion de la lumière de la peau et/ou des fibres kératiniques. Ces valeurs peuvent être comparées aux valeurs enregistrées dans la banque de données 55 et le centre de traitement 54 peut être configuré pour déterminer quel produit est le plus adapté à l'individu et pour envoyer ce résultat à l'ordinateur 53 par l'intermédiaire du réseau 52.

Dans un autre exemple de mise en oeuvre, on peut appliquer d'abord le produit sur un support, puis en saisir une première image à un premier instant et effectuer un premier calcul des coefficients de diffusion réduit et/ou d'absorption. On saisit ensuite une deuxième image à un deuxième instant, par exemple séparé du premier par un intervalle de temps d'environ une heure, et on effectue un deuxième calcul des coefficients de diffusion réduit et d'absorption du produit. Par comparaison de ces valeurs lors d'une étape d'évaluation, on peut déterminer l'évolution temporelle des valeurs caractéristiques de l'absorption et de la diffusion de la lumière.

On peut mesurer ainsi la rémanence d'un produit cosmétique par exemple.

On peut également réaliser, entre la première mesure et la seconde mesure, une action, par exemple pour ôter du produit. Le produit peut être par exemple un filtre solaire qu'on applique sur la peau et l'action précitée, une action de lavage. On peut ainsi déterminer la résistance à l'eau du filtre solaire, par exemple.

L'action exercée peut également être une action qui permet de modifier des propriétés physiques et/ou chimiques du produit et/ou du support, notamment la peau. On peut ainsi par exemple caractériser l'efficacité de la protection d'un filtre solaire en cours d'exposition aux rayons UV, par exemple en tenant compte des réactions de l'épiderme susceptibles d'interagir avec le produit.

Le produit peut également être un produit dépigmentant. On peut alors mesurer l'évolution dans le temps de la pigmentation de la peau après application du produit dépigmentant sur la peau, pour en caractériser l'efficacité.

Le produit peut encore être un produit antirides ou un produit hydratant.

On peut encore déterminer grâce à l'invention des valeurs caractéristiques de la diffusion et de l'absorption d'un produit appliqué sur un support inerte, sur la peau ou sur les fibres kératiniques, au cours de la mise au point de sa formulation, afin de déterminer l'influence de l'ajout de certains composants et modifier la formulation en conséquence, le cas échéant.

Bien entendu, l'invention n'est pas limitée aux exemples qui viennent d'être décrits.

Ainsi, on ne sort pas du cadre de la présente invention lorsqu'on calcule des valeurs représentatives de l'aptitude d'un produit à diffuser et/ou à absorber la lumière autres que les coefficients de diffusion réduit $\mu'_s$ et d'absorption $\mu_a$.

On peut encore combiner entre elles les caractéristiques des différents modes de réalisation décrits.

Dans toute la description, l'expression « comportant un » doit être comprise comme étant synonyme de « comportant au moins un », sauf si le contraire est spécifié.

**Revendications**

1. Procédé pour déterminer l'aptitude à diffuser et/ou à absorber la lumière

   - d'un produit cosmétique ou dermatologique ou
   - de ce produit cosmétique ou dermatologique appliqué sur un support, à l'exclusion de tout traitement thérapeutique du corps humain ou animal, **caractérisé par le fait qu'**il comporte les étapes suivantes :

   a) éclairer le produit et/ou une zone du support (S) sur laquelle a été appliqué le produit (P) avec un faisceau lumineux incident (20) de manière à former une tache de rétrodiffusion,
   b) acquérir une image de la tache, et
   c) analyser l'image pour déterminer, en fonction de la variation de la luminosité en différents points de la tache, au moins une information représentative de l'aptitude à diffuser la lumière et/ou à l'absorber du produit et/ou du support en présence du produit, à sawoir :

   - un coefficient de diffusion réduit $\mu'_s$ est déterminé en fonction de la variation de la luminosité en différents points de la tache situés à des distances différentes du centre de la tache,
   - un coefficient d'absorption de la lumière $m_a$ de la lumière est déterminé en fonction de la variation de la luminosité en différents points de la tache situés à des distances différentes du centre de la tache,

   le coefficient de diffusion réduit $\mu'_s$ et le coefficient d'absorption $\mu_a$ étant déterminés par le calcul de la réflectance R(r, φ) en fonction des coordonnées cylindriques, à partir du centre de la tache,

   en intégrant R (r, φ) en fonction de φ, on obtient la fonction $\overline{R}$ (r) :

   $$\overline{\overline{R}}(r) = \oint R(r, \varphi) \frac{d\varphi}{2\pi}$$

   la valeur de la réflectance $\overline{R}$ de la tache est intégrée en fonction du rayon r :

   $$Rint(r) = \int_0^r R(\rho)\, 2\,\pi\,\rho\,d\rho,$$

   une fonction du rayon est obtenue dont les constantes permettent d'accéder aux coefficients de diffusion et d'absorption :

   $$Rint(r) = a\left[1 - \exp\left(-\frac{r}{b}\right)\right]$$

   avec:

   $$a = a_1 \exp\left[-a_2\left[1 + \frac{\mu'_s}{\mu_a}\right]^{-0,5}\right]$$

   $$b = \left[b_1 + b_2 \ln\left(\frac{\mu_a}{\mu'_s}\right)\right]\mu'_s{}^{-1}$$

   et où $a_1$, $a_2$, $b_1$ et $b_2$ ne dépendent que de l'indice de réfraction n.

2. Procédé selon l'une quelconque des revendications précédentes, **caractérisé par le fait que** le support comporte des fibres et/ou des cellules kératiniques.

3. Procédé selon l'une quelconque des revendications précédentes, **caractérisé par le fait que** le support est inerte.

4. Procédé selon l'une quelconque des revendications précédentes, **caractérisé par le fait que** le support présente des reliefs.

5. Procédé selon l'une quelconque des revendications 1 à 3, **caractérisé par le fait que** la surface, éclairée par le faisceau lumineux incident, est sensiblement plante.

6. Procédé selon l'une quelconque des revendications précédentes, **caractérisé par le fait que** le produit est appliqué sur le support sur une superficie occupant entre 0,5 cm$^2$ et 5 cm$^2$, notamment entré 1 cm$^2$ et 2 cm$^2$.

7. Procédé selon l'une quelconque des revendications précédentes, **caractérisé par le fait que** le faisceau lumineux incident est un faisceau de lumière blanche.

8. Procédé selon l'une quelconque des revendications 1 à 6, **caractérisé par le fait que** le faisceau lumineux incident est un faisceau de lumière monochromatique.

9. Procédé selon l'une quelconque des revendications précédentes, **caractérisé par le fait que** l'intensité du faisceau lumineux incident est sensiblement constante sur toute sa section.

10. Procédé selon la revendication précédente, le faisceau lumineux incident étant produit par une source lumineuse, **caractérisé par le fait que** l'on dispose entre la source et le produit un filtre spatial.

11. Procédé selon l'une quelconque des revendications précédentes, **caractérisé par le fait que** l'angle d'incidence du faisceau lumineux incident avec la normale à la surface du produit est compris entre 5˚ et 25˚.

12. Procédé selon l'une quelconque des revendications précédentes, **caractérisé par le fait que** l'on dispose un miroir (33) sur le trajet du faisceau lumineux incident.

13. Procédé selon l'une quelconque des revendications précédentes, **caractérisé par le fait qu'**on acquiert l'image au moyen d'une caméra (40).

14. Procédé selon l'une quelconque des revendications précédentes, **caractérisé par le fait qu'**on transmet, l'image acquise à un centre de traitement (54), afin de l'analyser.

15. Procédé selon l'une quelconque des revendications précédentes, **caractérisé par le fait qu'**on acquiert l'image au travers d'un système optique ayant un grossissement variable.

16. Procédé selon l'une quelconque des revendications précédentes, le faisceau lumineux incident étant produit par une source lumineuse, **caractérisé par le fait qu'**un polariseur est disposé sur le trajet du faisceau lumineux incident, entre la source et le produit.

17. Procédé selon la revendication précédente, **caractérisé par le fait que** l'image est acquise au travers d'un analyseur.

18. Procédé selon l'une quelconque des revendications précédentes, **caractérisé par le fait que** l'image est acquise en l'absence de toute lumière parasite.

19. Procédé selon l'une quelconque des revendications précédentes, **caractérisé par le fait que** le faisceau lumineux incident est modulé et **par le fait que** l'acquisition de l'image est synchrone.

20. Procédé selon l'une quelconque des revendications précédentes, **caractérisé par le fait qu'**on applique le produit sur le support avec une épaisseur, comprise entre 7 $\mu$m et 20 $\mu$m.

21. Procédé selon l'une quelconque des revendications précédentes, **caractérisé par le fait que** le produit présente une composition homogène.

22. Procédé selon l'une quelconque des revendications précédentes, **caractérisé par le fait que** le produit est apte à modifier au moins une propriété physico-chimique du support.

23. Procédé selon l'une quelconque des revendications précédents, **caractérisé par le fait que** le produit est choisi parmi les produits suivants : fond de teint, filtre solaire, produit dépigmentant, produit anti-rides, produit hydratant.

24. Procédé pour déterminer une caractéristique d'un produit cosmétique ou dermatologique, à l'exclusion de tout traitement thérapeutique du corps humain ou animal, **caractérisé par le fait que** :

- l'on détermine l'aptitude à diffuser et/ou à absorber la lumière de deux surfaces composées respectivement

a) du produit appliqué sur la peau et/ou les fibres kératiniques, et
b) de la peau et/ou des fibres kératiniques en l'absence de produit, en mettant en oeuvre pour l'étape a) le procédé selon la revendication 1 et en réalisant pour b) :

i) éclairer une zone de la surface considérée avec un faisceau lumineux incident de manière à former une tache de rétrodiffusion,
ii) acquérir une image de la tache,
iii) analyser l'image pour déterminer, en fonction de la variation de la luminosité en différents points de la tache, au moins une information représentative de l'aptitude du produit, de la peau et/ou des fibres kératiniques à diffuser la lumière et/ou à l'absorber,

et **par le fait que** :

- l'on détermine en fonction des informations obtenues pour la peau et/ou les fibres kératiniques avec et sans produit au moins une caractéristique du produit.

25. Procédé pour déterminer l'évolution dans le temps d'une caractéristique d'un produit cosmétique ou dermatologique, à l'exclusion de tout traitement thérapeutique du corps humain ou animal, **caractérisé par le fait qu'**il comporte les étapes suivantes :

a) appliquer le produit (P) sur un support (S),
b) mettre en oeuvre le procédé selon la revendication 1 sur le support sur laquelle le produit a été appliqué à un premier instant,
c) réaliser le procédé selon la revendication 1 à un second instant,
d) déterminer en fonction des informations obtenues lors des étapes b) et c) l'évolution dans le temps d'une caractéristique du produit.

26. Procédé selon la revendication précédente, **caractérisé par le fait qu'**il comporte l'étape suivante, entre les étapes b) et c) :

- exercer une action sur le produit et/ou le support, notamment une action de lavage ou d'exposition au vent ou à un rayonnement UV ou de contact avec un élément sur lequel le produit est susceptible de se transférer.

27. Procédé selon l'une des revendications 25 ou 26, **caractérisé par le fait que** la caractéristique du produit qui est déterminée est sa couvrance.

28. Procédé selon l'une des revendications 25 ou 26, le produit étant un filtre solaire, **caractérisé par le fait que** la caractéristique qui est déterminée est l'efficacité de la protection conférée par le produit en fonction du temps.

29. Procédé selon l'une des revendications 25 ou 26, le produit étant un produit dépigmentant, **caractérisé par le fait que** la caractéristique qui est déterminée est l'efficacité du produit.

30. Dispositif de détermination de l'aptitude d'un produit cosmétique ou dermatologique à diffuser et/ou à absorber la lumière, **caractérisé par le fait qu'**il comporte :

- une source (31) apte à délivrer un faisceau lumineux (20) capable de former une tache lumineuse sur le produit ou sur un support (S) sur lequel a été appliqué le produit (P),
- une caméra, permettant d'acquérir au moins une image de la tache,
- un dispositif d'analyse (50) pour analyser cette image et déterminer, en fonction de la variation de la luminosité en différents points de la tache, à des distances différentes du centre de la tache, au moins une information représentative de l'aptitude du produit à diffuser la lumière et/ou à l'absorber
- un coefficient de diffusion réduit $\mu'_s$ étant déterminé en fonction de la variation de la luminosité en différents points de la tache situés à des distances différentes du centre de la tache;
- un coefficient d'absorption de la lumière $m_a$ de la lumière étant déterminé en fonction de la variation de la luminosité en différentes points de la tache situés à des distances différentes du centre de la tache,

le coefficient de diffusion réduit $\mu'_s$ et le coefficient d'absorption $\mu_a$ étant déterminé par le calcul de la réflectance $R(r, \varphi)$
en fonction des coordonnées cylindriques, à partir du centre de la tache,

en intégrant $R(r, \varphi)$ en fonction de $\varphi$, on obtient la fonction $\overline{R}(r)$ :

$$\overline{R}(r) = \oint R(r, \varphi) \frac{\mathrm{d}\varphi}{2\pi}$$

la valeur de la réflectance $\overline{R}$ de la tache étant intégrée en fonction du rayon $r$ :

$$\mathrm{Rint}(r) = \int_0^r R(\rho)\, 2\,\pi\,\rho\,\mathrm{d}\rho,$$

une fonction du rayon étant obtenue dont les constantes permettent d'accéder aux coefficients de diffusion et d'absorption :

$$\mathrm{Rint}(r) = a\left[1 - \exp\left(-\frac{r}{b}\right)\right]$$

avec:

$$a = a_1 \exp\left[-a_2\left(1 + \frac{\mu'_s}{\mu_a}\right)^{-0,5}\right]$$

$$b = \left[ b_1 + b_2 \ln\left(\frac{\mu_a}{\mu'_s}\right) \right] {\mu'_s}^{-1}$$

et où $a_1$, $a_2$, $b_1$ et $b_2$ ne dépendent que de l'indice de réfraction n.

**31.** Dispositif selon la revendication précédente, **caractérisé par le fait que** la source lumineuse comporte un laser.

**32.** Dispositif selon l'une des deux revendications précédentes, **caractérisé par le fait qu'**il comporte un filtre spatial (32) traversé par le faisceau lumineux.

**33.** Dispositif selon l'une quelconque des revendications 30 à 32, **caractérisé par le fait qu'**il comporte un miroir (33) placé sur le trajet du faisceau lumineux.

**34.** Dispositif selon l'une quelconque des revendications 30 à 33, **caractérisé par le fait que** le dispositif d'analyse (50) est configuré pour recevoir l'image de la caméra par un réseau.

**35.** Procédé de fabrication d'un produit cosmétique ou dermatologique, **caractérisé par le fait qu'**il comporte les étapes suivantes :

a) réaliser le procédé selon la revendication 1 sur une zone de la peau pour déterminer, au moins une information représentative de l'aptitude de la peau à diffuser la lumière et/ou à l'absorber,

b) fabriquer, au moins en fonction de cette information, un produit cosmétique ou dermatologique.

**Claims**

**1.** A method of determining the capacity for diffusing and/or absorbing light

- of a cosmetic or dermatological substance; or
- of said cosmetic or dermatological substance applied on a support, excluding any therapeutic treatment of the human or animal body, the method being **characterized by** the fact that it comprises the following steps:

a) illuminating the substance and/or a zone of the support (S) on which the substance (P) has been applied with an incident light beam (20) in such a manner as to form a back-scatter spot;

b) acquiring an image of the spot; and

c) analyzing the image as a function of variation in brightness between the various points of the spot to determine at least one item of information representative of the capacity for diffusing light and/or absorbing it of the substance and/or the support in the presence of the substance, namely:

- a reduced diffusion coefficient $\mu'_s$ is determined as a function of the variation in the brightness at various points of the spot, situated at different distances from the center of the spot; and
- a light absorption coefficient $m_a$ is determined as a function of the variation in the brightness at various points of the spot, situated at different distances from the center of the spot;

the reduced diffusion coefficient $\mu'_s$ and the absorption coefficient $\mu_a$ being determined by calculating reflectance $R(r,\varphi)$ as a function of cylindrical coordinates starting from the center of the spot, integrating $R(r,\varphi)$ as a function of $\varphi$, the function $\overline{R}(r)$ is obtained:

$$\overline{R}(r) = \oint R(r, \varphi) \frac{d\varphi}{2\pi}$$

the value of the reflectance R of the spot is integrated as a function of radius $\underline{r}$:

$$R\,int(r) = \int_0^r R(\rho)2\pi\rho.d\rho$$

a function of the radius is obtained having constants which give access to diffusion and absorption coefficients:

$$R\,int(r) = a\left[1 - exp\left(-\frac{r}{b}\right)\right]$$

with

$$a = a_1\,exp\left[-a_2\left(1 + \frac{\mu'_s}{\mu_a}\right)^{-0.5}\right]$$

$$b = \left[ b_1 + b_2 \ ln\left(\frac{\mu_a}{\mu'_s}\right)\right]\mu'_s{}^{-1}$$

and where $a_1$, $a_2$, $b_1$, and $b_2$ depend only on refractive index $\underline{n}$.

2. A method according to any preceding claim, **characterized by** the fact that the support comprises keratinous fibers and/or cells.

3. A method according to any preceding claim, **characterized by** the fact that the support is inert.

4. A method according to any preceding claim, **characterized by** the fact that the support presents relief.

5. A method according to any one of claims 1 to 3, **characterized by** the fact that the surface illuminated by the incident light beam is substantially plane.

6. A method according to any preceding claim, **characterized by** the fact that the substance is applied on the support over an area in the range 0.5 cm$^2$ to 5 cm$^2$, and in particular in the range 1 cm$^2$ to 2 cm$^2$.

7. A method according to any preceding claim, **characterized by** the fact that the incident light beam is a beam of white light.

8. A method according to any one of claims 1. to 6, **characterized by** the fact that the incident light beam is a beam of monochromatic light.

9. A method according to any preceding claim, **characterized by** the fact that the intensity of the incident light beam is substantially constant over its entire section.

10. A method according to the preceding claim, in which the incident light beam is produced by a light source and **characterized by** the fact that a spatial filter is placed between the light source and the substance.

11. A method according to any preceding claim, **characterized by** the fact that the angle of incidence of the incident light beam relative to the normal to the surface of the substance lies in the range 5° to 25°

12. A method according to any preceding claim, **characterized by** the fact that a mirror (33) is placed on the path of the incident light beam.

13. A method according to any preceding claim, **characterized by** the fact that the image is acquired by means of a camera (40).

14. A method according to any preceding claim, **characterized by** the fact that the acquired image is transmitted to a processing center (54) in order to be analyzed.

15. A method according to any preceding claim, **characterized by** the fact that the image is acquired through an optical system of variable magnification.

16. A method according to any preceding claim, in which the incident light beam is produced by a light source, the method being **characterized by** the fact that a polarizer is placed on the path of the incident light beam between the source and the substance.

17. A method according to the preceding claim, **characterized by** the fact that the image is acquired through an analyzer.

18. A method according to any preceding claim, **characterized by** the fact that the image is acquired in the absence of any interfering light.

19. A method according to any preceding claim, **characterized by** the fact that the incident light beam is modulated and by the fact that image acquisition is synonymous.

20. A method according to any preceding claim, **characterized by** the fact that the substance is applied on the support with a thickness lying in the range 7 $\mu$m to 20 $\mu$m.

21. A method according to any preceding claim, **characterized by** the fact that the substance is of uniform composition.

22. A method according to any preceding claim, **characterized by** the fact that the substance is suitable for modifying at least one physico-chemical property of the support.

23. A method according to any preceding claim, **characterized by** the fact that the substance is selected from the following: foundation makeup, sunscreen, depigmenting cream, antiwrinkle cream, moisturizer.

24. A method of determining a characteristic of a cosmetic or dermatological substance, excluding any therapeutic treatment of the human or animal body, the method being **characterized by** the fact that it comprises the steps of:

   - determining the capacities of two surfaces to diffuse and/or absorb light, the surfaces being constituted respectively:

a) by a substance applied to the skin and/or the keratinous fibers; and

b) by the skin and/or keratinous fibers in the absence of the substance, by implementing for step a) the method according to claim 1 and by performing for step b) :

i) illuminating a zone of the surface in question with an incident light beam in such a manner as to form a back-scatter spot;

ii) acquiring an image of the spot; and

iii) analyzing the image as a function of variation in brightness at various points of the spot in order to determine at least one item of information representative of the capacity of the substance, the skin, and/or the keratinous fibers to diffuse light and/or to absorb it;

and:

- determining at least one characteristic of the substance as a function of the information obtained for the skin and/or the keratinous fibers with and without the substance.

25. A method of determining the way a characteristic of a cosmetic or dermatological substance varies over time, excluding any therapeutic treatment of the animal or human body, the method being **characterized by** the fact that it comprises the following steps:

a) applying the substance (P) on a support (S);

b) at a first instant, implementing the method according to claim 1 on the support on which the substance has been applied;

c) at a second instant, performing the method according to claim 1; and

d) determining the variation over time of a characteristic of the substance as a function of information obtained during steps b) and c)

26. A method according to the preceding claim, **characterized by** the fact that it includes the following step between steps b) and c):

- exercising an action on the substance and/or the support, in particular a washing action or exposure to the wind or to UV radiation or making contact with an element on which the substance is to be transferred.

27. A method according to claim 25 or claim 26, **characterized by** the fact that the characteristic of the substance which is determined is its covering ability.

28. A method according to claim 25 or claim 26, in which the substance is a sunscreen, the method being **characterized by** the fact that the characteristic which is determined is the effectiveness of the protection conferred by the substance as a function of time.

29. A method according to claim 25 or claim 26, in which the substance is depigmenting cream, **characterized by** the fact that the characteristic which is determined is the effectiveness thereof.

30. Apparatus for determining the capacity of a cosmetic or dermatological substance to diffuse and/or absorb light, the apparatus being **characterized by** the fact that it comprises:

- a source (31) suitable for delivering a light beam (20) capable of forming a light spot on the substance or on a support (S) on which a substance (P) has been applied;

- a camera enabling at least one image of the spot to be acquired; and

- an analyzer apparatus (50) for analyzing said image as a function of variation in brightness at various points of the spot at different distances from the center of the spot, to determine at least one item of information representative of the capacity of the substance to diffuse light and/or to absorb it:

- a reduced diffusion coefficient $\mu'_s$ being determined as a function of the variation in the brightness at various points of the spot, situated at different distances from the center of the spot;

- a light absorption coefficient $m_a$ being determined as a function of the variation in the brightness at various points of the spot, situated at different distances from the center of the spot;

the reduced diffusion coefficient $\mu'_s$ and the absorption coefficient $\mu_a$ being determined by calculating reflectance $R(r,\varphi)$ as a function of cylindrical coordinates starting from the center of the spot, integrating $R(r,\varphi)$ as a function of $\varphi$, the function $\overline{R}(r)$ is obtained:

$$\overline{R}(r) = \oint R(r, \varphi)\,\frac{d\varphi}{2\pi}$$

the value of the reflectance $\overline{R}$ of the spot being integrated as a function of radius $\underline{r}$:

$$R\,int(r) = \int_0^r R(\rho)2\pi\rho.d\rho$$

a function of the radius being obtained having constants which give access to diffusion and absorption coefficients:

$$R\,int\,(r) = a\left[1 - exp\left(-\frac{r}{b}\right)\right]$$

with

$$a = a_1\,exp\left[-a_2\left(1 + \frac{\mu'_s}{\mu_a}\right)^{-0.5}\right]$$

$$b = \left[b_1 + b_2\,ln\left(\frac{\mu_a}{\mu'_s}\right)\right]\mu'_s{}^{-1}$$

and where $a_1$, $a_2$, $b_1$, and $b_2$ depend only on refractive index $\underline{n}$.

31. Apparatus according to the preceding claim, **characterized by** the fact that the light source comprises a laser.

32. Apparatus according to either one of the two preceding claims, **characterized by** the fact that it includes a spatial filter (32) through which the light beam passes.

33. Apparatus according to any one of claims 30 to 32, **characterized by** the fact that it includes a mirror (33) placed on the path of the light beam.

34. Apparatus according to any one of claims 30 to 33 **characterized by** the fact that the analysis apparatus (50) is configured to receive the camera image via a network.

35. A method of preparing a cosmetic or dermatological substance, the method being **characterized by** the fact that it comprises the following steps:

  a) performing the method according to claim 1 on a zone of the skin to determine at least one item of information representative of the capacity of the skin to diffuse light and/or to absorb it; and
  b) at least as a function of said information, preparing a cosmetic or dermatological substance.

**Patentansprüche**

1. Verfahren zum Bestimmen der Fähigkeit zum Streuen und/oder zum Absorbieren des Lichtes

  - eines kosmetischen oder dennatologischen Produktes oder
  - von diesem kosmetischen oder dermatologischen Produkt, das auf einen Träger appliziert ist, unter Ausschluss von sämtlichen therapeutischen Behandlungen eines menschlichen oder tierischen Körpers, **dadurch gekennzeichnet, dass** dieses die folgenden Schritte aufweist.

  a) das Produkt und/oder ein Bereich des Trägers (S), auf welchem das Produkt (P) appliziert worden ist, wird mit einem einfallenden Lichtbündel (20) bestrahlt, in der Weise, um einen Ruckstreuungsfleck auszubilden,
  b) erhalten eines Bildes des Flecks, und
  c) Analysieren des Bildes, um als Funktion der Änderung der Helligkeit an verschiedenen Punkten des Flecks zumindest eine Information zu bestimmen, die die Fähigkeit zum Streuen des Lichtes und/oder zum Absorbieren durch das Produkt und/oder den Träger in Anwesenheit des Produkts repräsentiert, um zu erfahren:

  - ein reduzierter Streukoeffizient $\mu'_s$ wird als Funktion der Änderung der Leuchtstärke bzw. Helligkeit an verschiedenen Punkten des Flecks, die in verschiedenen Abständen von dem Mittelpunkt des Flecks angeordnet sind, bestimmt,
  - ein Absorptionskoeffizient des Lichtes $m_a$ des Lichtes wird als Funktion der Änderung der Leuchtstärke an verschiedenen Punkten des Flecks, die in verschiedenen Abständen zu dem Mittelpunkt des Flecks angeordnet sind, bestimmt,

wobei der reduzierte Streukoeffizient $\mu'_s$ und der Absorptionskoeffizient $\mu_a$ durch Berechnung des Reflektionsvermögens $R(r,\varphi)$ als Funktion der Zylinderkoordinaten ab dem Zentrum des Fleckes bestimmt werden,
wobei $R(r, \varphi)$ als Funktion von $\varphi$ integriert wird, wobei die Funktion $\overline{R}(r)$:

$$\overline{R}(r) = \oint R(r,\varphi)\frac{d\varphi}{2\pi}$$

wobei der Wert des Reflektionsvermögens $\overline{R}$ des

Flecks als Funktion des Strahls r integriert wird:

$$\mathrm{Rint}(r) = \int_0^r R(\rho)\, 2\,\pi\,\rho\, d\rho,$$

wobei eine Funktion des Strahls erhalten wird, von dem die Konstanten ermöglichen, Zugang zu dem Koeffizienten der Streuung und der Absorption zu erhalten:

$$\mathrm{Rint}(r) = a\left[1 - \exp\left(-\frac{r}{b}\right)\right]$$

mit

$$a = a_1 \exp\left[-a_2\left(1 + \frac{\mu'_s}{\mu_a}\right)^{-0.5}\right]$$

$$b = \left[b_1 + b_2 \ln\left(\frac{\mu_a}{\mu'_s}\right)\right]{\mu'_s}^{-1}$$

und wo $a_1$, $a_2$, $b_1$ und $b_2$ nur abhängig sind von dem Brechungsindex n.

2. Verfahren nach einem der voranstehenden Ansprüche, **dadurch gekennzeichnet, dass** der Träger Fasern und/oder keratinischen Zellen aufweist.

3. Verfahren nach irgendeinem der voranstehenden Ansprüche, **dadurch gekennzeichnet, dass** der Träger inert bzw. neutral ist.

4. Verfahren nach irgendeinem der voranstehenden Ansprüche, **dadurch gekennzeichnet, dass** der Träger Erhöhungen vorzuweisen hat

5. Verfahren nach irgendeinem der Ansprüche 1 bis 3, **dadurch gekennzeichnet, dass** die Oberfläche, die von dem einfallenden Lichtbündel bestrahlt wird, genau eben ist.

6. Verfahren nach irgendeinem der voranstehenden Ansprüche, **dadurch gekennzeichnet, dass** das Produkt auf dem Träger auf eine Oberfläche appliziert ist, die zwischen 0,5 cm$^2$ und 5 cm$^2$, insbesondere zwischen 1 cm$^2$ und 2 cm$^2$ besetzt.

7. Verfahren nach irgendeinem der voranstehenden Ansprüche, **dadurch gekennzeichnet, dass** das einfallende Lichtbündel ein Bündel weißen Lichts ist.

8. Verfahren nach irgendeinem der Ansprüche 1 bis 6, **dadurch gekennzeichnet, dass** das einfallende Lichtbündel ein Bündel von monochromatischem Licht ist.

9. Verfahren nach irgendeinem der voranstehenden Ansprüche, **dadurch gekennzeichnet, dass** die Intensität des einfallenden Lichtbündels über seinen ganzen Bereich genau konstant ist.

10. Verfahren nach dem voranstehenden Anspruch, wobei das einfallende Lichtbündel durch eine Leuchtquelle erzeugt wird, **dadurch gekennzeichnet, dass** zwischen der Quelle und dem Produkt ein räumlicher Filter angeordnet wird.

11. Verfahren nach irgendeinem der voranstehenden Ansprüche, **dadurch gekennzeichnet, dass** der Einfallswinkel des einfallenden Lichtbündels zu der Normalen an der Oberfläche des Produktes zwischen 5˚ und 25˚ liegt.

12. Verfahren nach irgendeinem der voranstehenden Ansprüche, **dadurch gekennzeichnet, dass** ein Spiegel (33) auf der Trajektorie des einfallenden Lichtbündels angeordnet wird.

13. Verfahren nach irgendeinem der voranstehenden Ansprüche, **dadurch gekennzeichnet, dass** das Bild mittels einer Kamera (40) erhalten wird.

14. Verfahren nach irgendeinem der voranstehenden Ansprüche, **dadurch gekennzeichnet, dass** das Bild, das an einem Mittelpunkt der Behandlung (54) erhalten wird, übertragen wird, um es zu analysieren.

15. Verfahren nach irgendeinem der voranstehenden Ansprüche, **dadurch gekennzeichnet, dass** das Bild durch ein optisches System erhalten wird, das eine variable Vergrößerungskraft hat.

16. Verfahren nach irgendeinem der voranstehenden Ansprüche, wobei das einfallende Lichtbündel mittels einer Leuchtquelle erzeugt wird, **dadurch gekennzeichnet, dass** eine Polarisationseinrichtung auf der Trajektorie des einfallenden Lichtbündels zwischen der Quelle und dem Produkt angeordnet ist.

17. Verfahren nach dem voranstehenden Anspruch, **dadurch gekennzeichnet, dass** das Bild nach Durchquerung eines Analysators erhalten wird.

**18.** Verfahren nach irgendeinem der voranstehenden Ansprüche, **dadurch gekenntzeichnet, dass** das Bild in Abwesenheit allen parasitären Lichts erhalten wird.

**19.** Verfahren nach irgendeinem der voranstehenden Ansprüche, **dadurch gekennzeichnet, dass** das einfallende Lichtbündel moduliert wird und **dadurch**, dass die Erlangung des Bildes synchron erfolgt.

**20.** Verfahren nach irgendeinem der voranstehenden Ansprüche, **dadurch gekennzeichnet, dass** das Produkt auf dem Träger in einer Dicke appliziert wird, die zwischen 7 µm und 20 µm liegt.

**21.** Verfahren nach irgendeinem der voranstehenden Ansprüche, **dadurch gekenntzeichnet, dass** das Produkt eine homogene Zusammensetzung darstellt.

**22.** Verfahren nach irgendeinem der voranstehenden Ansprüche, **dadurch gekennzeichnet, dass** das Produkt dazu in der Lage ist, zumindest eine physikalisch-chemische Eigenschaft des Trägers zu verändern.

**23.** Verfahren nach irgendeinem der voranstehenden Ansprüche, **dadurch gekennzeichnet, dass** das Produkt unter den folgenden Produkten ausgewählt wird: Teintgrundierung bzw. Make-up, Sonnenfilter, Depigmentierungsprodukt, Antifalteiiprodukt, Hydrata.üonsprodujkt.

**24.** Verfahren zum Bestimmen einer Charakteristik eines kosmetischen oder dermatologischen Produkts, mit Ausnahme von sämtlichen therapeutischen Behandlungen des menschlichen oder tierischen Körpers, **dadurch gekennzeichnet, dass:**

    - die Fähigkeit zum Streuen und/oder zum Absorbieren des Lichtes von zwei Oberflächen bestimmt wird, die jeweils zusammengesetzt sind,

        a) einem auf der Haut und/oder keratinische Fasern applizierten Produkt, und
        b) von Haut und/oder keramischen Fasern in Abwesenheit des Produkts, wobei für den Schritt a) von dem Verfahren nach dem Anspruch 1 Gebrauch gemacht wird, und wobei für b) verwirklicht wird:

            i) ein Bereich der in Betracht gezogenen Oberfläche wird mit einem einfallenden Lichtbündel in der Weise bestrahlt, um einen Rückstremmgsfleck auszubilden,
            ii) ein Bild des Flecks wird erhalten,
            iii) das Bild wird analysiert, um als Funktion der Veränderung der Leuchtkraft an verschiedenen Punkten des Flecks zumindest eine Information zu bestimmen, die für die Fähigkeit des Produktes, von der Haut und/oder den keratinische Fasern, das Licht zu streuen und/oder zu absorbieren, zu bestimmen,

und **dadurch**:

    - dass als Funktion der erhaltenen Information, die für die Haut und/oder die keratinischen Fasern mit und ohne Produkt erhalten worden ist, zumindest eine Charakteristik des Produkts bestimmt wird.

**25.** Verfahren zum Bestimmen einer zeitlichen Entwicklung einer Charakteristik eines kosmetischen oder dermatologischen Produktes, mit Ausnahme sämtlicher therapeutischer Behandlungen des menschlichen oder tierischen Körpers, **dadurch gekennzeichnet, dass** es die folgenden Schritte enthält:

    a) das Produkt (P) wird auf einen Träger (S) appliziert,
    b) das Verfahren nach Anspruch 1 wird auf dem Träger, auf dem das Produkt in einem ersten Moment appliziert worden ist, angewendet,
    c) in einem zweiten Moment wird das Verfahren nach dem Anspruch 1 durchgeführt,
    d) als Funktion der Informationen, die während der Schritte b) und c) erhalten worden sind, wird die zeitliche Entwicklung einer Charakteristik des Produktes bestimmt.

**26.** Verfahren nach dem voranstehenden Anspruch, **dadurch gekennzeichnet, dass** es den nachfolgenden Schritt zwischen den Schritten b) und c) aufweist:

    - eine Aktion wird auf das Produkt und/oder den Träger ausgeübt, insbesondere eine Aktion des Spülens oder des Aussetzens gegenüber Wind oder einer UV-Strahlung oder des Kontaktes zu einem Element bei welchem das Produkt eine Neigung aufweist, sich zu übertragen.

**27.** Verfahren nach einem der Ansprüche 25 oder 26, **dadurch gekenntzeichnet, dass** die Charakteristik des Produktes, welche bestimmt wird, seine Deckfähigkeit ist.

**28.** Verfahren nach einem der Ansprüche 25 oder 26, wobei das Produkt ein Sonnenfilter ist, **dadurch gekennzeichnet, dass** die Charakteristik, welche bestimmt wird, die Wirksamkeit des Schutzes ist, die durch das Produkt als Funktion der Zeit bereitgestellt wird.

**29.** Verfahren nach einem der Ansprüche 25 oder 26, wobei das Produkt ein depigmentierendes Produkt ist, **dadurch gekennzeichnet, dass** die Charakteristik, die bestimmt wird, die Wirksamkeit bzw. Leistungsfähigkeit des Produktes ist.

**30.** Vorrichtung zur Bestimmung der Fähigkeit eines kosmetischen oder dermatologischen Produktes, Licht zu streuen und/oder zu absorbieren, **dadurch gekennzeichnet, dass** diese aufweiset:

- eine Quelle (31), die dazu in der Lage ist, ein Lichtbündel (20) zu liefern, das dazu in der Lage ist, einen Leuchtfleck auf dem Produkt oder auf einem Träger (S), auf welchen das Produkt (P) appliziert ist, zu erzeugen,
- eine Kamera, die es erlaubt, zumindest ein Bild des Flecks zu erhalten,
- eine Analysevorrichtung (50), um dieses Bild zu analysieren und als Funktion der Veränderung der Leuchtkraft an verschiedenen Punkten des Fleckes in verschiedenen Abständen zu der Mitte des Flekkes zumindest eine Information zu bestimmen, die für die Fähigkeit des Produktes zum Streuen und/oder zum Absorbieren des Lichtes repräsentativ ist,
- einen reduzierten Streukoeffizienten $\mu'_s$, der als Funktion der Veränderung der Leuchtstärke an verschiedenen Punkten des Fleckes, die in verschiedenen Abständen zu der Mitte des Flekkes angeordnet sind, bestimmt wird,
- einen Lichtabsorptionskoeffizienten $m_a$ des Lichtes, der als Funktion der Veränderung der Leuchtkraft an verschiedenen Punkten des Fleckes, die in verschiedenen Abständen zu der Mitte des Flecks positioniert sind, bestimmt wird,

wobei der reduzierte Streukoeffizient $\mu'_s$ und der Absorptionskoeffizient $\mu_a$ durch die Berechnung des Reflektionsvermögens $R(r, \varphi)$ als Funktion von Zylinderkoordinaten ab dem Zentrum des Flecks bestimmt werden,
indem $R(r, \varphi)$ als Funktion von $\varphi$ integriert wird, wobei die Funktion $\overline{R}(r)$ erhalten wird:

$$\overline{R}(r) = \oint R(r, \varphi) \frac{d\varphi}{2\pi}$$

wobei der Wert des Reflektionsvermögens $\overline{R}$ des Flecks als Funktion des Strahls r integriert wird:

$$\mathrm{Rint}(r) = \int_0^r R(\rho)\, 2\pi \rho\, d\rho,$$

wobei eine Funktion des Strahls erhalten wird, von dem die Konstanten es erlauben, auf Streu- und Absorptionskoeffizienten zuzugreifen:

$$\mathrm{Rint}(r) = a\left[1 - \exp\left(-\frac{r}{b}\right)\right]$$

mit

$$a = a_1 \exp\left[-a_2\left(1 + \frac{\mu'_s}{\mu_a}\right)^{-3,3}\right]$$

$$b = \left[b_1 + b_2 \ln\left(\frac{\mu_a}{\mu'_s}\right)\right]\mu'_s{}^{-1}$$

und wo $a_1$, $a_2$, $b_1$ und $b_2$ nur von dem Brechungsindex n abhängen.

**31.** Vorrichtung nach dem voranstehenden Anspruch, **dadurch gekennzeichnet, dass** die Leuchtquelle einen Laser aufweiset.

**32.** Vorrichtung nach einem der zwei voranstehenden Ansprüche, **dadurch gekennzeichnet, dass** diese einen räumlichen Filter (32) aufweist, der von dem Lichtbündel durchquert wird.

**33.** Vorrichtung nach irgendeinem der Ansprüche 30 bis 32, **dadurch gekennzeichnet, dass** diese einen Spiegel (33) aufweist, der auf der Trajektorie des Lichtbündels angeordnet ist.

**34.** Vorrichtung nach irgendeinem der Ansprüche 30 bis 33, **dadurch gekennzeichnet, dass** die Analysevorrichtung (50) konstruiert ist, um das Bild von der Kamera durch ein Gitter zu empfangen.

**35.** Verfahren zur Herstellung eines kosmetischen oder dermatologischen Produktes, **dadurch gekennzeichnet, dass** dieses die folgenden Schritte aufweist:

a) das Verfahren nach Anspruch 1 wird auf einem Bereich von der Haut realisiert, um zumindest eine Information zu bestimmen, die für die Fähigkeit der Haut zum Streuen und/oder zum Absorbieren des Lichtes repräsentativ ist,

b) ein kosmetisches oder dennatologisches Produkt wird zumindest als Funktion von dieser Information hergestellt.

FIG.1

FIG.2

FIG.3

FIG.4

FIG.5

FIG.6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1156074 A

- US 5923039 A